# EUROPEAN PATENT SPECIFICATION

(11) **EP 1 653 885 B1**
(45) Date of publication and mention of the grant of the patent: **01.04.2009**
(21) Application number: 04780838.1
(22) Date of filing: 12.08.2004
(51) Int. Cl.: A61F 2/06

(54) **DYNAMIC STENT**
DYNAMISCHER STENT
STENT DYNAMIQUE

(30) Priority: 12.08.2003 US 494476 P; 30.04.2004 US 835826
(43) Date of publication of application: 10.05.2006
(73) Proprietor: Failure Analysis of Cardiovascular Technologies, Inc., Gulf Stream, FL 33483 (US)
(72) Inventor: MOORE, James, E., Jr., College Station, TX 77845 (US); MORENO, Michael, R., College Station, TX 77845 (US)
(74) Representative: Wright, Howard Hugh Burnby
(86) International application number: PCT/US2004/026064
(87) International publication number: WO 2005/018489

(56) References cited:
- WO-A-00/44309
- WO-A-02/074198
- US-A- 5 342 348
- US-A1- 2002 082 680
- US-A1- 2003 135 265
- US-B1- 6 258 117
- US-B1- 6 569 193

## Description

### FIELD OF THE INVENTION

The present invention relates generally to stents, and more particularly to stents providing dynamic support of a vessel after implantation.

### BACKGROUND OF THE INVENTION

Stenting is a non-surgical treatment used with balloon angioplasty to treat coronary artery disease. Right after angioplasty has widened a coronary artery, a *stent* (one example being a small, expandable wire mesh tube) is inserted within the artery. The purpose of the stent is to help hold the newly treated artery open, reducing the risk of the artery re-closing (restenosis) over time.

Although stents have been widely used as solid mechanical, structural supports to maintain a vessel in a non-collapsed state following balloon angioplasty, they are not without their problems. Studies on the response of the artery wall to a stent demonstrate that the artery wall responds in distinct phases, displaying varying behaviors during certain time intervals after implantation. The earliest response, thrombus formation, is followed by ramping up inflammatory responses, smooth muscle cell proliferation, and finally, remodeling. Re-endothelization of the intima occurs on the time frame of weeks. Research has demonstrated that stent design influences these actions through biomechanically mediated responses. For example, blood flow patterns dictate that platelet deposition is lowest when stent strut spacing is small, whereas endothelial cell regrowth is fastest when stent strut spacing is large. Stent-induced artery wall stresses (which depend heavily on strut configuration) also play a role in the inflammatory and proliferative responses. While each of these responses have distinctly different characteristic times of action, previously developed stents are either static and do not change over time, or are fully degradable and may fail to provide sufficient structural support for supporting the artery. Thus, there exists a need for a stent which is reliable, easy to manufacture, and which is dynamic such that the properties of the stent change over time to correspond to the changing responses and needs of the vessel.

US 2003/0135265 (Stinson) discloses a body-insertable tubular stent including discrete tubular segments in an alternating sequence of segments having high axial stiffness and segments having low axial stiffness. The lower axial stiffness segments are intended for placement along more severely curved regions of the body vessel, to provide a greater degree of stent conformity to the vessel. The more axially flexible segments can be provided by winding a metallic or polymeric strand at a higher pitch along such segments, thus forming a higher braid angle in a braided stent. In such cases the more axially flexible segments also exert a higher radial force when the stent is radially compressed. Alternatively, the stent can consist of multiple interbraided strands, with each strand incorporating a higher number of filaments (at least two) along axially stiff segments, and incorporating a lower number of filaments (at least one) along axially flexible segments. In another alternative version, the device is formed of one or more resilient strands, each strand including a biostable filament and a bioabsorbable filament, so that after implantation the radial force and axial stiffness gradually decrease *in vivo.*

### SUMMARY OF THE INVENTION

One embodiment of a stent formed in accordance with the present invention is disclosed. The stent, for deployment in a vessel, comprises an elongated support frame for supporting a vessel in a non-collapsed state, characterized by the support frame including the combination of a plurality of circumferentially spaced, longitudinally extending support struts; a plurality of first durable annular links resistant to degradation over time extending circumferentially of, spaced apart lengthwise of and connected to the support struts, and a plurality of degradable annular links extending circumferentially of, spaced apart lengthwise of and connected to the support struts, at least some of the degradable links being positioned between first links to assist in support of a vessel in a non-collapsed state after implementation, the degradable links being constructed and arranged to degrade after implementation over a predetermined time such that support provided by the degradable links decreases a selected amount over a predetermined time after implantation, such that the mechanical characteristics of the stent and link spacing change over time after implantation.
Various advantageous features are set out in the claims.

### BRIEF DESCRIPTION OF THE DRAWINGS

The foregoing aspects and many of the attendant advantages of this invention will become better understood by reference to the following detailed description, when taken in conjunction with the accompanying drawings, wherein:
FIGURE 1 is a perspective view of one embodiment of a dynamic stent formed in accordance with the present invention, the dynamic stent including a permanent component and a temporary component;
FIGURE 2 is a side view of the dynamic stent of FIGURE 1;
FIGURE 3 is a perspective view of the permanent component of the dynamic stent of FIGURE 1;
FIGURE 4 is a side view of the permanent component of the dynamic stent of FIGURE 1;
FIGURE 5 is a perspective view of the temporary component of the dynamic stent of FIGURE 1;
FIGURE 6 is a side view of the temporary component of the dynamic stent of FIGURE 1; and
FIGURE 7 is a side view of an alternate embodiment of a dynamic stent formed in accordance with the present invention, wherein the dynamic stent is formed so as to have a variable stiffness along the length of the dynamic stent.

### DETAILED DESCRIPTION OF THE PREFERRED EMBODIMENT

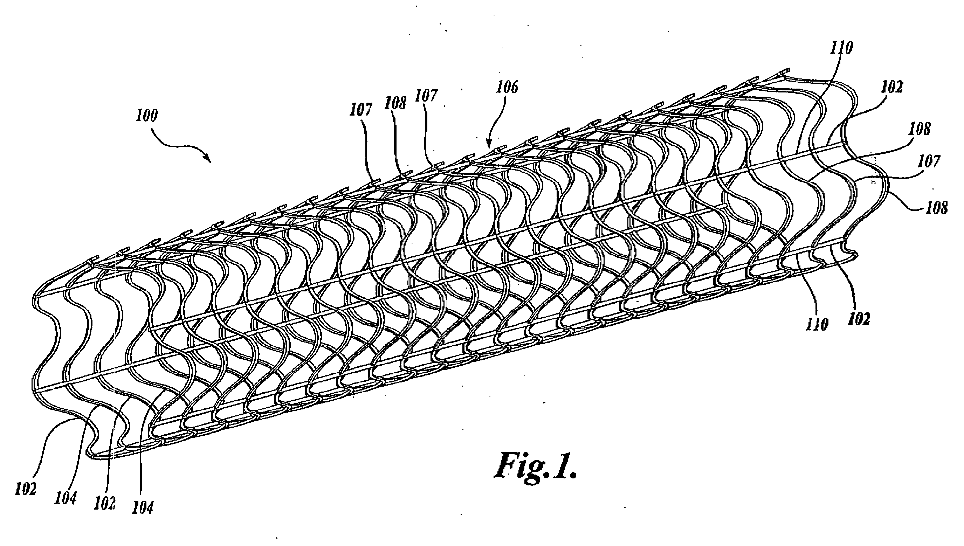

Referring to FIGURES 1-6, one embodiment of a dynamic stent 100 formed in accordance with the present invention is depicted. The dynamic stent 100 incorporates a hybrid structure having both a durable or permanent component 102 and a temporary component 104. The permanent component 102 is suitably a durable structure that remains in the artery for an extended period, such as for the life of the user. The temporary component 104 changes over time to accommodate the changing requirements of artery wall rehabilitation after stent implantation.

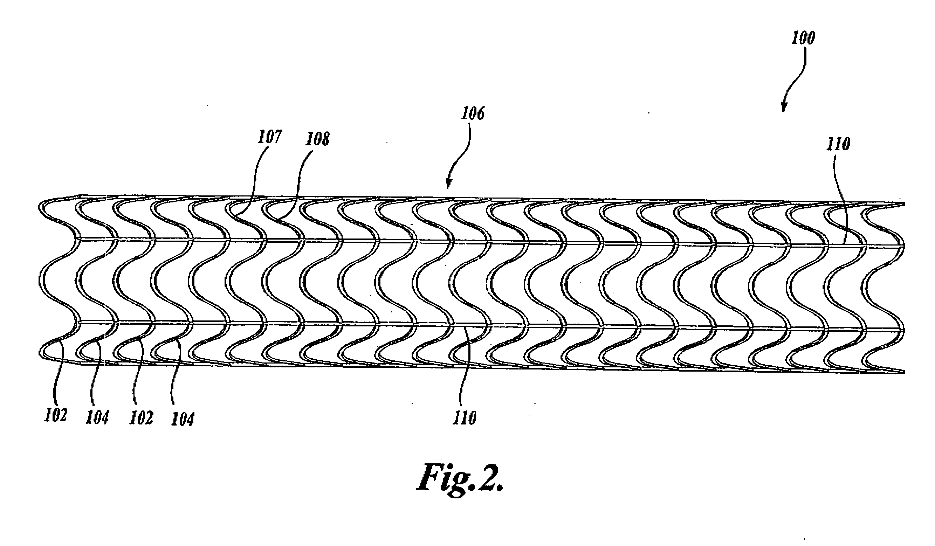

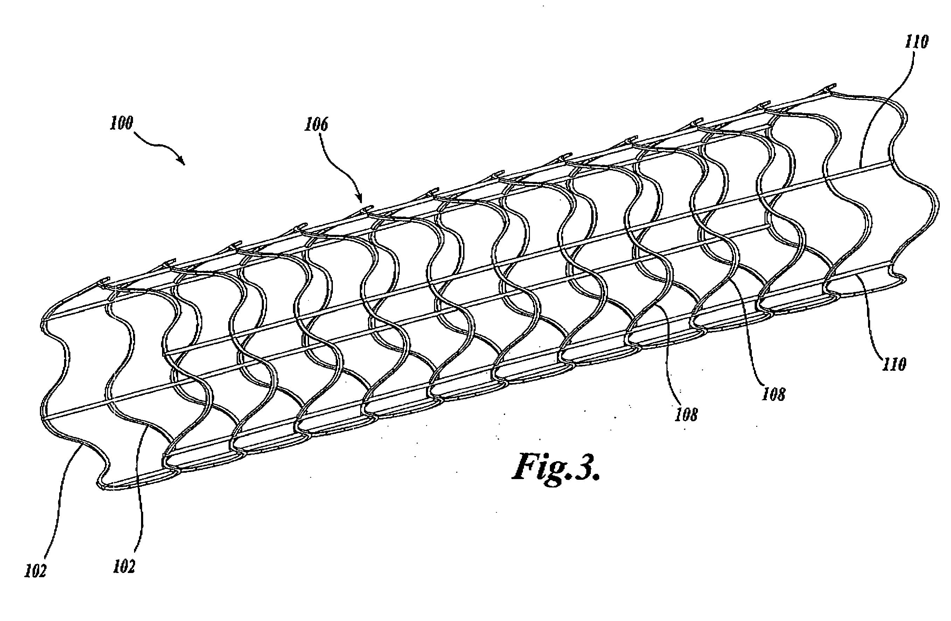

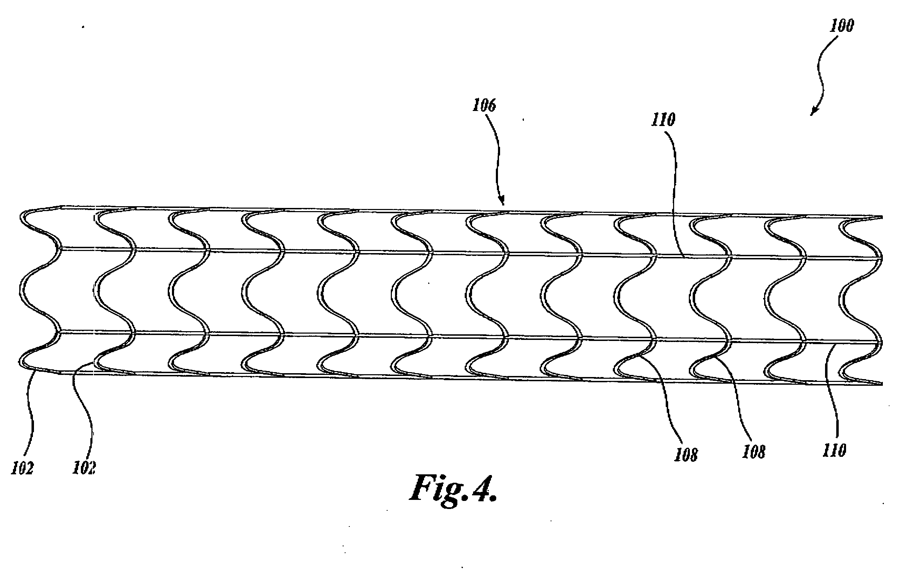

Moreover, in the early stages after implantation, both the permanent and temporary components 102 and 104 are present in the dynamic stent 100, such as shown in FIGURES 1 and 2, providing improved artery wall support through small strut spacing. This configuration serves to minimize platelet deposition and to hold back intimal flaps. As time passes, the temporary component 104 degrades away, eventually leaving just the permanent component 102, as shown in FIGURES 3 and 4. In this new phase of artery rehabilitation, the dynamic stent 100 has a rather sparse strut spacing which increases the shear stress on the artery wall, since shear stress depends heavily on strut spacing. This larger strut spacing is allowable because there is presumably less of a need to hold back intimal flaps once partial healing of the artery wall has occurred.

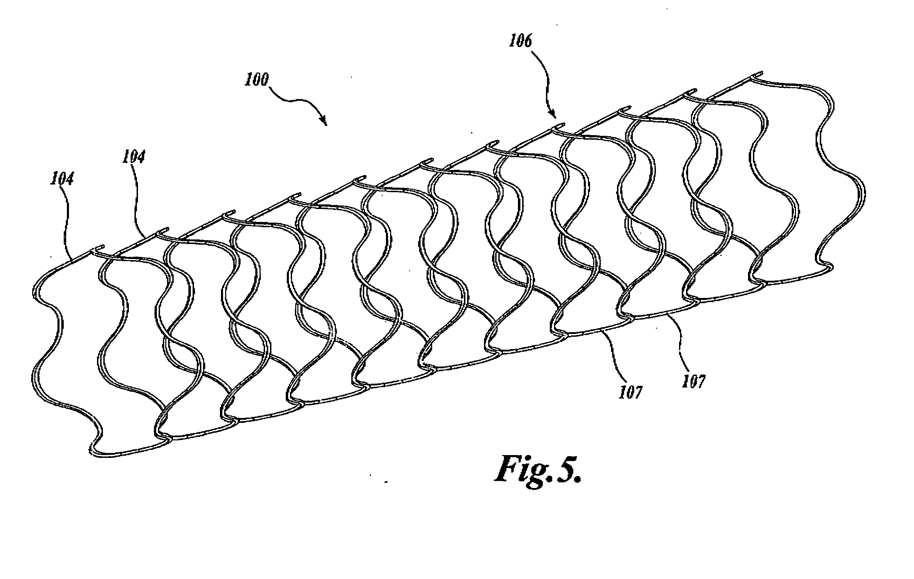

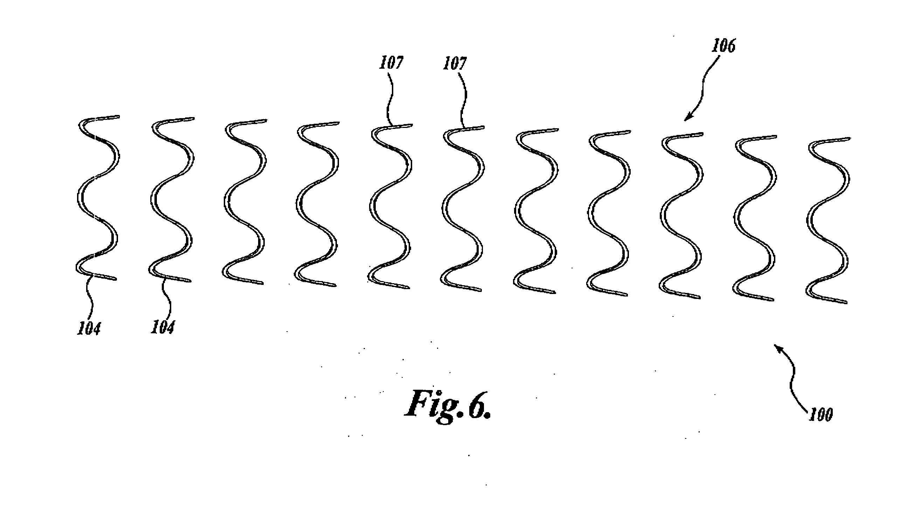

Referring to FIGURES 1 and 2, this detailed description will now focus upon the structure of the dynamic stent 100. As stated above, the dynamic stent 100 includes a permanent component 102 (best shown in FIGURES 3 and 4) and a temporary component 104 (best shown in FIGURES 5 and 6). The permanent and temporary components 102 and 104 are interwoven with one another, and in combination, form a support frame 106 for supporting an artery wall (not shown). The support frame 106 is tubular in shape providing a central lumen along its central axis.

The support frame 106 includes a plurality of struts or annular first and second links 107, 108. The annular links 107, 108 of the illustrated embodiment are sinusoidal in shape and are generally equally spaced along the length of the dynamic stent 100. The sinusoidal shape of the annular links 107, 108 provides a blunt end profile for the dynamic stent 100 in order minimize the risk of puncturing the vessel and provides increased support of the artery wall over a straight annular link. Further, the sinusoidal shape of the annular links 107, 108 permits the dynamic stent 100 to be expanded from a small diameter to a larger diameter once the dynamic stent is properly positioned within the artery. The dynamic stent 100 may be expanded by any suitable technique, such as by balloon expansion or self expansion.

Although a sinusoidal shape of the annular links 107, 108 is described and depicted, it should be apparent to those skilled in the art that other shapes of the links are suitable for use with the present invention, some suitable examples being links formed from repeating geometric shapes, such as triangles, squares, circles, polygons, arcuate shapes, parabolic shapes, oval shapes, linear shapes, and non-sinusoidal shapes. In the illustrated embodiment, the dynamic stent 100 includes a series of twenty-three (23) annular links 107, 108 spaced equidistant from one another along the longitudinal length of the dynamic stent 100. The annular links 107, 108 are spaced from one another such that adjacent annular links are disposed in a nested relationship relative to one another, such that a crest of the sinusoidal wave of one annular link is nested at least partially between a pair of troughs of the sinusoidal wave of an adjacent annular link .

The spacing of the annular links 107, 108 from one another is maintained by an array of longitudinally oriented struts 110. The struts 110 are linear in form and are preferably oriented parallel with the longitudinal axis of the dynamic stent 100. The struts 110 pass through and are connected to each of the annular links . In the illustrated embodiment, six (6) longitudinal struts 110 are used, spaced equidistant from each other about the circumference of the dynamic strut 100. Further, although a specific number, orientation, and shape of the longitudinal struts 110 is described and depicted, it should be apparent to those skilled in the art that alternate numbers, orientations, and shapes of longitudinal struts 110 are suitable for use with and within the spirit and scope of the present invention. Although the longitudinally oriented struts 110 are depicted and described as extending continuously from one end of the dynamic stent 100 to the other, it should be apparent to those skilled in the art that the longitudinally oriented struts 110 may be intermittently disposed along the length of the dynamic stent 100, such as to connect only a few annular links to one another.

As mentioned above, the dynamic stent 100 includes a permanent component 102 and a temporary component 104 which are interwoven/interlaced with one another, and in combination, to form the support frame 106. Referring to FIGURES 3 and 4, the dynamic stent 100 is depicted with the temporary component removed, leaving solely the permanent component 102. The permanent component 102 includes the longitudinal struts 110 and every other one of the annular links, namely durable links 108 of the support frame 106. Moreover, the permanent component 102 includes twelve (12) of the durable annular links 108, including the annular links 108 disposed at the ends of the dynamic stent 100.

The permanent component 102 is formed from any suitable rigid or semi-rigid material which is resistant to degradation in the body and which is compatible with the human body and bodily fluids that the dynamic stent 100 may contact. Further, preferably the dynamic stent 100 should be made from a material that allows for expansion of the dynamic stent 100 and which is able to retain its expanded shape while disposed within the lumpen of the body passage. A few examples of suitable materials include stainless steel, tantalum, titanium, chromium cobalt, and nitinol.

The permanent component 102 may be formed using traditional techniques such as laser machining of tube stock, Electrical Discharge Machining (EDM), etc. The permanent component 102 may be self-expanding or balloon expandable. As should be apparent to those skilled in the art, a relatively sparse mesh pattern for the permanent component 102 may provide benefit with regard to delivery profile. A less dense mesh pattern for the agent component 102 may mean that a ratio of a first collapsed diameter to a second expanded diameter may be smaller.

Referring to FIGURES 5 and 6, the dynamic stent 100 depicted in FIGURES 1 and 2 is shown with the permanent component removed, leaving solely the temporary component 104. The temporary component 104 includes every other one of the annular links, namely, temporary or degradable links 107 of the support frame 106. Moreover, the temporary component 104 includes eleven (11) of the annular link 107, each of the annular links 107, of the temporary component being sandwiched between a pair of adjacent annular links 108 of the permanent component.

The temporary component 104 is formed from any suitable rigid or semi-rigid material which is amenable to degradation in the body and which is compatible with the human body and bodily fluids that the dynamic stent 100 may contact. Further, preferably the temporary component 100 is made from a material that allows for expansion of the dynamic stent 100 and which is able to retain its expanded shape while disposed within the lumen of the body passage. A few examples of suitable materials include polymers, such as the polylactide (PLA) polymer or the polymers disclosed in U.S. Patent No. 6,461,631, the disclosure of which is hereby expressly incorporated by reference, hydrogels, and magnesium alloys. The material used may include therapeutic substances which are selectively released once the dynamic stent is implanted to aid rehabilitation of the artery wall, one such suitable material disclosed in U.S. Patent No. 6,506,437, the disclosure of which is hereby expressly incorporated by reference.

The temporary component 104 may be formed on the permanent component 102 by dipping the permanent component 102 in a liquid polymer. The liquid polymer is then cured upon the permanent component 102. The dynamic stent 100 may then be made into custom shapes by selective physical cutting and removal of certain pieces. Other selective removal techniques may be used as well, such as laser machining. Preferably, the permanent and temporary components 102 and 104 are each formed in a geometric array, mesh; chain, interlinking pattern, etc. Preferably; the permanent and temporary components 102 and 104 are coupled to one another, such as by interconnecting and/or interweaving one to the other. Alternately, the meshwork of polymer may also be produced by lining the inside and/or outside of the permanent component 102 with a weave of polymer fibers.

In still another alternate embodiment, the temporary component 104 may be a substantially complete covering, rather than a meshwork. In still yet another embodiment, the temporary component 104 may be a substantially complete covering made of a porous, biodegradable material. The porosity may come from laser machining, from physical hole punching, or from other traditional techniques of making porous polymers.

Preferably, the temporary component 104 is formed from a biodegradable mesh of sufficient density to hold back intimal flaps and other wall/plaque components that have intruded into the lumen. The need to prop these flaps against the wall likely goes away after partial healing; presumably occurring on the order of weeks after implantation. In light of the above description of the structure of the dynamic stent 100, the use of the dynamic stent 100 will now be described. The dynamic stent 100 is inserted within a blood vessel using well mown techniques. The permanent component 102 and temporary component 104 may be delivered together into the blood vessel. Alternately, the permanent component 102 would be delivered, and then the temporary component 104 would be extruded into the artery via a catheter approach. The extrusion geometry may be a standard geometry, or a custom geometry based on the plaque geometry and composition, as imaged by intravascular ultrasound or optical coherence tomography.

As time after implant progresses, the temporary component 104 preferably degrades, resulting in a stent geometry that adjusts over time to match the changing needs of the artery wall during the remodeling process. When initially inserted, both the permanent and temporary components 102 and 104 are fully present, as shown in FIGURES 1 and 2. As time after implantation increases, the temporary component 104 degrades, resulting in the dynamic stent 100 eventually taking the form shown in FIGURE 3 and 4, wherein the temporary component 104 is absent. The temporary component 104 may be embedded with drugs to modulate cellular reactions, a few example being to modulate smooth muscle cell proliferation, inflammatory responses, and/or thrombus formation.

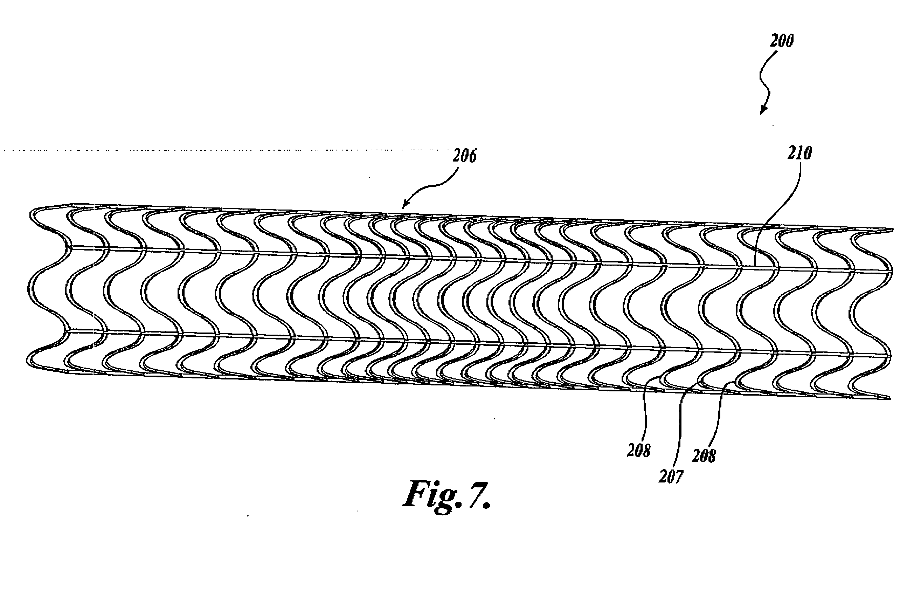

Referring to FIGURE 7, an alternate embodiment of a dynamic stent 200 formed in accordance with the present invention is shown. The dynamic stent 200 is identical to the dynamic stent 100 described and depicted above with relation to FIGURES 1-6 with the exception that the stiffness of the dynamic stent 200 is variable along a length of the dynamic stent 200. In the illustrated embodiment, this accomplished by providing a support frame 206 that is non-uniform in shape. For instance, in the illustrated embodiment, the structure of the support frame 206 is modified so as to be non-uniform along its length by adjusting the spacing of the annular links 207, 208 forming the support frame 206 in combination with the longitudinal support struts 210 by which the annular links are supported. More specifically, the spacing of the annular links 208 near the midpoint of the dynamic stent 200 is less than the spacing of the annular links 208 at the ends of the dynamic stent. Thus, the dynamic stent has a stiffness that is variable along the length of the dynamic stent 200, such that the stiffness at a pair of ends of the dynamic stent 200 is less than at the midpoint of the dynamic stent 200.

Referring to FIGURE 2, the dynamic stent 100 depicted therein may be modified to provide variable stiffness along a length of the dynamic stent 100. This may be accomplished by forming the degradable component 104 from a plurality of degradable components, each degradable component having a different rate of degradation. Thus, in one embodiment, the dynamic stent has a uniform stiffness along the length of the dynamic stent upon insertion into the blood vessel. However the degradable component 104 is formed from a high rate degradable material at the ends of the dynamic stent 100 and a low rate degradable material near the midpoint of the dynamic stent 100. After implantation, the annular links 107 of the degradable component 104 disposed at the ends of the dynamic stent 100 degrade at an elevated rate and accordingly disappear first. The annular links 107 of the degradable component 104 located at the midpoint of the dynamic stent 100 degrade at a slower rate, and therefore remain in place for a longer duration. This variable degradation of the degradable component 104 results in a variable stiffness of the support frame 106 along a longitudinal length of the support frame 106 such that a stiffness at a pair of ends of the support frame 106 is less than a stiffness at a .. middle of the support frame 106 after a selected period after implantation.

Although this detailed description depicts and describes two separate embodiments, wherein in one, materials of different degradation rates are used to provide variable stiffness characteristics and wherein in a second, the spacing/shape of the support frame is modified to provide variable stiffness characteristics, it should be apparent that combinations thereof are within the spirit and scope of the present invention.

While the preferred embodiment of the invention has been illustrated and described, it will be appreciated that various changes can be made therein without departing from the spirit and scope of the invention.

## Claims

1. A stent (100; 200) for deployment in a vessel, said stent comprising an elongated support frame (106; 206) for supporting a vessel in a non-collapsed state, **characterized by** the support frame (106; 206) including the combination of:
(a) a plurality of circumferentially spaced, longitudinally extending support struts (110; 210);
(b) a plurality of first durable annular links (108; 208) resistant to degradation over time, extending circumferentially of, spaced apart lengthwise of and connected to the support struts (110; 210), and
(c) a plurality of degradable annular links (107; 207) extending circumferentially of, spaced apart lengthwise of and connected to the support struts (110; 210); at least some of the degradable links (107; 207) being positioned between first links (108; 208) to assist in support of a vessel in a non-collapsed state after implementation, the degradable links (107; 207) being constructed and arranged to degrade after implementation over a predetermined time such that support provided by the degradable links (107; 207) decreases a selected amount over a predetermined time after implantation, such that the mechanical characteristics of the stent and link spacing change over time after implantation.

2. The stent of Claim 1, further **characterized by** degradation of the degradable links (107; 207) resulting in a variable stiffness over time, after implantation of the stent, along the longitudinal length of the stent.

3. The stent of Claim 1, further **characterized by** the support struts (110; 210) and first annular links (108; 208) being durable and resistant to degradation over time.

4. The stent of any preceding claim, further **characterized by** degradation of the degradable links (107; 207) resulting in a variable stiffness over time, after implantation of the stent, along the longitudinal length of the stent.

5. The stent of any preceding claim, further **characterized by** the degradable links (107; 207) being degradable over time such that after a predetermined duration the surface area of the support frame exposed to the vessel is decreased.

6. The stent of any preceding claim, further **characterized by** the first links (107; 207) and degradable links (108; 208) alternating along the length of the stent.

7. The stent of claim 6, further **characterized by** the degradable links (108; 208) being degradable to the extent that the degradable links are substantially eliminated from the support frame, thereby increasing spacing retaining adjacent first links.

8. The stent of any preceding claim, further **characterized by** the first and degradable links being disposed in a nested relationship.

9. The stent of any preceding claim, further **characterized by** the first links and the degradable links being substantially sinusoidal shaped.

## Patentansprüche

1. Stent (100; 200) zum Einsatz in ein Blutgefäß, wobei der Stent einen länglichen Stützrahmen (106; 206) zur Stützung eines Blutgefäßes in nicht kollabiertem Zustand umfasst, **dadurch gekennzeichnet, dass** der Stützrahmen (106; 206) folgende Kombination aufweist:
a) eine Mehrzahl am Umfang entlang voneinander beabstandeter, in Längsrichtung verlaufender Stützstreben (110; 210);
b) eine Mehrzahl erster haltbarer ringförmiger Glieder (108; 208), die nicht im Laufe der Zeit biologisch abgebaut werden und die am Umfang der Stützstreben (110; 210) entlang verlaufen, in Längsrichtung zu diesen Stützstreben (110; 210) voneinander beabstandet sowie mit diesen verbunden sind; und
c) eine Mehrzahl abbaubarer ringförmiger Glieder (107; 207), die am Umfang der Stützstreben (110; 210) entlang verlaufen, in Längsrichtung zu diesen Stützstreben (110; 210) voneinander beabstandet sowie mit diesen verbunden sind; wobei zumindest einige dieser abbaubaren Glieder (107; 207) als zusätzliche Stützung eines Blutgefäßes in nicht kollabiertem Zustand nach Implementation zwischen ersten Gliedern (108; 208) angeordnet sind, wobei die abbaubaren Glieder (107; 207) derart konstruiert und angeordnet sind, dass sie sich nach der Implementation über eine vorbestimmte Zeit hinweg zersetzen, so dass die Stützwirkung der abbaubaren Glieder (107; 207) in ausgewähltem Umfang über eine vorbestimmte Zeitspanne nach der Implantation abnimmt, wodurch sich die mechanischen Eigenschaften des Stent sowie der Abstand zwischen den Gliedern im Laufe der Zeit nach der Implantation verändern.

2. Stent nach Anspruch 1, weiterhin **dadurch gekennzeichnet, dass** sich infolge des Zerfalls der abbaubaren Glieder (107; 207) die Steife des Stent im Laufe der Zeit nach dessen Implantation über seine Länge in Längsrichtung hinweg verändert.

3. Stent nach Anspruch 1, weiterhin **dadurch gekennzeichnet, dass** die Stützstreben (110; 210) und die ersten ringförmigen Glieder (108; 208) haltbar und gegen Zerfall im Laufe der Zeit resistent sind.

4. Stent nach einem der vorhergehenden Ansprüche, weiterhin **dadurch gekennzeichnet, dass** sich infolge des Zerfalls der abbaubaren Glieder (107; 207) die Steife des Stent im Laufe der Zeit nach dessen Implantation über seine Länge in Längsrichtung hinweg verändert.

5. Stent nach einem der vorhergehenden Ansprüche, weiterhin **dadurch gekennzeichnet, dass** die abbaubaren Glieder (107; 207) im Laufe der Zeit biologisch abgebaut werden können, so dass nach einer vorbestimmten Zeitdauer die dem Blutgefäß ausgesetzte Fläche des Stützrahmens abnimmt.

6. Stent nach einem der vorhergehenden Ansprüche, weiterhin **dadurch gekennzeichnet, dass** die ersten Glieder (108; 208) und die abbaubaren Glieder (107; 207) über die Länge des Stent hinweg abwechselnd vorgesehen sind.

7. Stent nach Anspruch 6, weiterhin **dadurch gekennzeichnet, dass** die abbaubaren Glieder (108; 208) in einem derartigen Umfang abgebaut werden können, dass sie im Wesentlichen aus dem Stützrahmen eliminiert werden, wodurch sich der zwischen benachbarten ersten Gliedern verbleibende Abstand vergrößert.

8. Stent nach einem der vorhergehenden Ansprüche, weiterhin **dadurch gekennzeichnet, dass** die ersten Glieder und die abbaubaren Glieder in verschachtelter Anordnung vorgesehen sind.

9. Stent nach einem der vorhergehenden Ansprüche, weiterhin **dadurch gekennzeichnet, dass** die ersten Glieder und die abbaubaren Glieder im Wesentlichen sinusförmig ausgebildet sind.

## Revendications

1. Stent (100 ; 200) pour déploiement dans un vaisseau, ledit stent comprenant un treillis de support allongé (106 ; 206) pour soutenir un vaisseau dans un état non effondré, **caractérisé en ce que** le treillis de support (106 ; 206) inclut la combinaison de :
(a) une pluralité de tiges de support (110 ; 210) s'étendant longitudinalement, espacées sur la circonférence ;
(b) une pluralité de premières liaisons annulaires durables (108 ; 208), résistant à la dégradation dans le temps, s'étendant de façon circonférentielle et espacées les unes des autres sur la longueur des tiges de support (110 ; 210) et connectées à ces dernières, et
(c) une pluralité de liaisons annulaires dégradables (107 ; 207) s'étendant de façon circonférentielle et espacées les unes des autres sur la longueur des tiges de support (110 ; 210) et connectées à ces dernières ; certaines au moins des liaisons dégradables (107 ; 207) étant positionnées entre les premières liaisons (108 ; 208) pour aider au support d'un vaisseau dans un état non affaissé après mise en place, les liaisons dégradables (107 ; 207) étant construites et disposées pour se dégrader après mise en place en une durée prédéterminée telle que le support assuré par les liaisons dégradables (107 ; 207) diminue d'un niveau choisi sur une durée prédéterminée après implantation, de sorte que les caractéristiques mécaniques du stent et de l'espacement de liaison changent dans le temps après implantation.

2. Stent selon la revendication 1, **caractérisé en outre par** la dégradation des liaisons dégradables (107 ; 207) entraînant une rigidité variable dans le temps, après implantation du stent, le long de la longueur longitudinale du stent.

3. Stent selon la revendication 1, **caractérisé en outre par le fait que** les tiges de support (110 ; 210) et les premières liaisons annulaires (108 ; 208) sont durables et résistantes à la dégradation dans le temps.

4. Stent selon la revendication précédente, **caractérisé en outre par le fait que** la dégradation des liaisons dégradables (107 ; 207) entraîne une rigidité variable dans le temps, après implantation du stent, le long de la longueur longitudinale du stent.

5. Stent selon la revendication précédente, **caractérisé en outre par le fait que** les liaisons dégradables (107 ; 207) sont dégradables dans le temps de sorte qu'après une durée prédéterminée, la surface du treillis de support exposée au vaisseau est diminuée.

6. Stent selon la revendication précédente, **caractérisé en outre par le fait que** les premières liaisons (107 ; 207) et les liaisons dégradables (108 ; 208) alternent le long de la longueur du stent.

7. Stent selon la revendication 6, **caractérisé en outre en ce que** les liaisons dégradables (108 ; 208) sont dégradables au point où les liaisons dégradables sont pour l'essentiel éliminées du treillis de support, augmentant ainsi l'espacement restant entre les premières liaisons adjacentes.

8. Stent selon la revendication précédente, **caractérisé en outre en ce que** les premières liaisons et les liaisons dégradables sont disposées dans une relation imbriquée.

9. Stent selon la revendication précédente, **caractérisé en outre en ce que** les premières liaisons et les liaisons dégradables sont pour l'essentiel de forme sinusoïdale.
